(19) Europäisches Patentamt
European Patent Office
Office européen des brevets

(11) Veröffentlichungsnummer: **0 346 620 A1**

(12) **EUROPÄISCHE PATENTANMELDUNG**

(21) Anmeldenummer: **89108715.7**

(51) Int. Cl.4: **C07D 249/10 , A01N 25/32**

(22) Anmeldetag: **16.05.89**

Patentansprüche für folgenden Vertragsstaat: ES.

(30) Priorität: **20.05.88 DE 3817192**

(43) Veröffentlichungstag der Anmeldung:
**20.12.89 Patentblatt 89/51**

(84) Benannte Vertragsstaaten:
**DE ES FR GB IT**

(71) Anmelder: **HOECHST AKTIENGESELLSCHAFT**
**Postfach 80 03 20**
**D-6230 Frankfurt am Main 80(DE)**

(72) Erfinder: **Löher, Heinz Josef, Dr.**
**Amselweg 9**
**D-6238 Hofheim am Taunus(DE)**
Erfinder: **Heubach, Günther, Dr.**
**Luisenstrasse 15**
**D-6233 Kelkheim (Taunus)(DE)**
Erfinder: **Bauer, Klaus, Dr.**
**Doorner Strasse 54D**
**D-6450 Hanau(DE)**
Erfinder: **Bieringer, Hermann, Dr.**
**Eichenweg 26**
**D-6239 Eppstein/Taunus(DE)**

(54) **1,2,4-Triazolderivate enthaltende pflanzenschützende Mittel sowie neue Derivate des 1,2,4-Triazols.**

(57) Die vorliegende Erfindung betrifft pflanzenschützende Mittel zum Einsatz als Safener für Herbizide, welche eine Triazolverbindung der Formel I oder deren Salz enthalten,

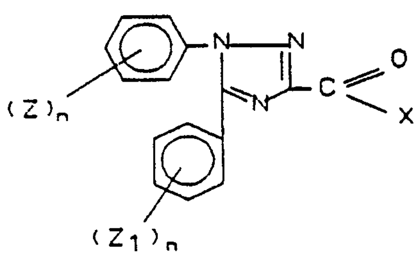

( I )

worin

Z, Z1 Halogen, Nitro, Cyano, einen gegebenenfalls substituierten Amino-, Alkyl-, Alkoxy-, Alkylthio-, Cycloalkyl-, Phenyl- oder Phenoxy-Rest,

X Hydroxy, $-OCH_2Si(CH_3)_3$, Alkyl, Cycloalkoxy, Phenoxy, Alkenyloxy, Alkinyloxy, (subst.) Alkoxy, (subst.) Alkylthio, einen Rest der Formeln

EP 0 346 620 A1

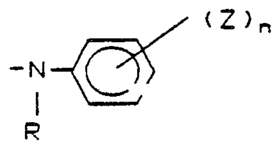 , 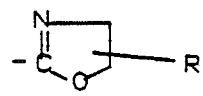

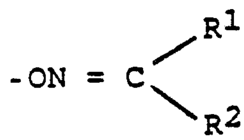 oder 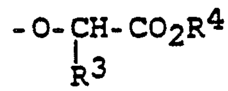

und n = 0, 1, 2 oder 3 bedeuten.

Eine große Zahl der Verbindungen der Formel I ist neu. Diese Verbindungen und deren Herstellung sind ebenfalls Gegenstand der Erfindung.

## 1,2,4-Triazolderivate enthaltende pflanzenschützende Mittel sowie neue Derivate des 1,2,4-Triazols

Bestimmte phenylsubstituierte 1,2,4-Triazolderivate sind bereits als Safener zum Schutz von Kulturpflanzen gegen phytotoxische Nebenwirkungen von Herbiziden bekannt (EP-A-0174562 oder US-A 4,639,266). Es wurde gefunden, daß andere Triazolderivate mit zwei Phenylsubstituenten am Triazolring vorteilhafte Eigenschaften als Safener besitzen.

Die vorliegende Erfindung betrifft daher pflanzenschützende Mittel, welche eine Triazolverbindung der Formel I oder deren Salz enthalten,

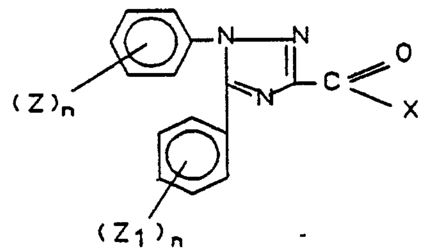

(I)

worin

Z und $Z_1$ unabhängig voneinander Halogen, Nitro, Cyano, $(C_1$-$C_4)$-Alkyl, $(C_1$-$C_4)$Alkoxy, $(C_1$-$C_4)$-Alkylthio, wobei die Alkyl-, Alkoxy- und Alkylthiogruppen durch ein oder mehrere Halogenatome, insbesondere Fluor oder Chlor, substituiert sein können, $(C_3$-$C_6)$Cycloalkyl, das durch $(C_1$-$C_4)$Alkyl substituiert sein kann, Amino, $(C_1$-$C_4)$-Alkylamino, $(C_1$-$C_4)$-Dialkylamino, Hydroxymethyl, $(C_1$-$C_4)$Alkoxymethyl, wobei die Alkyl- und Alkoxygruppen in den vorgenannten Resten durch $(C_1$-$C_4)$Alkyl substituiert sein können, Phenyl oder Phenoxy, wobei Phenyl und Phenoxy durch Halogen ein- oder mehrfach und/oder einfach durch Trifluormethyl substituiert sein können,

X Hydroxy, $-OCH_2Si(CH_3)_3$, $(C_1$-$C_4)$Alkyl, $(C_3$-$C_6)$Cycloalkoxy, Phenyl$(C_1$-$C_6)$alkoxy, Phenoxy, $(C_2$-$C_6)$-Alkenyloxy, $(C_2$-$C_6)$-Alkinyloxy, $(C_1$-$C_6)$Alkoxy, $(C_1$-$C_6)$Alkylthio, wobei die Alkoxy oder Alkylthiogruppe ein- oder zweifach durch $(C_1$-$C_2)$Alkoxy, Mono- oder Di- $(C_1$-$C_4)$Alkyl-phenylaminocarbonyl, Mono- oder Di- $(C_1$-$C_6)$Alkylamino, $(C_1$-$C_6)$Alkyl-carbonyloxy, $(C_1$-$C_2)$Alkylthio, Cyano oder ein-oder mehrfach durch Halogen substituiert sein kann, einen Rest der Formeln

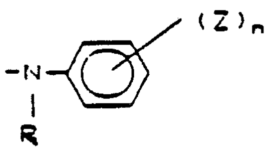

oder

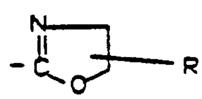

worin jeweils R Wasserstoff oder $(C_1$-$C_4)$Alkyl bedeutet, ferner Mono- oder Di- $(C_1$-$C_4)$Alkylamino, $(C_5$-$C_6)$-Cycloalkylamino, Piperidino, Morpholino oder 2,6-Dimethylmorpholino, einen Rest der Formel

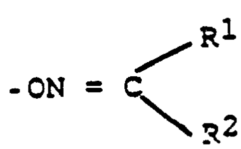

worin

$R^1$ und $R^2$ unabhängig voneinander $(C_1$-$C_4)$Alkyl bedeuten oder $R^1$ und $R^2$ gemeinsam mit dem sie verknüpfenden C-Atom einen 5-, 6- oder 7-gliedrigen Cycloalkylrest bilden können, ferner einen Rest der Formel

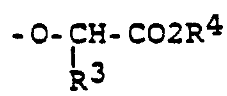

worin $R^3$ und $R^4$ unabhängig voneinander H oder $(C_1\text{-}C_4)$Alkyl, bedeuten, und n unabhängig voneinander die Zahl 0, 1, 2 oder 3 bedeutet.

Im Falle X = OH können die Verbindungen der Formel I Salze bilden. Erfindungsgemäß einsetzen lassen sich die in der Landwirtschaft verwendbaren Salze. Als solche kommen beispielsweise Metallsalze wie Alkali- oder Erdalkalisalze, insbesondere Natrium-oder Kaliumsalze, Salze mit Ammonium, Mono-, Di-, Tri- oder Tetra- $(C_1\text{-}C_4)$alkylammonium oder mit Mono-, Di-, Tri- oder Tetra- $(C_1\text{-}C_4)$alkanolammonium infrage.

Ferner erfaßt Formel I auch alle Stereoisomeren und deren Gemische. Diese treten auf, wenn X ein Rest, der ein asymmetrisches C-Atom enthält, bedeutet.

Unter den genannten Verbindungen der Formel I sind diejenigen bevorzugt, bei denen
Z, $Z_1$ = Cl, F, $(C_1\text{-}C_4)$Alkoxy, $(C_1\text{-}C_4)$Alkyl oder $CF_3$,
X = $(C_1\text{-}C_6)$Alkoxy oder Hydroxy und
n = 0, 1, 2 oder 3 bedeuten.

Einige der Verbindungen der Formel I sind aus wissenschaftlichen Veröffentlichungen (Synthesis 1985, 3, 304-305; Synthesis 1986, 9, 772-774 sowie aus JP-A 065312; Chem. and Ind. 1960, Nr. 34, 1085; Canad. J. Chem. 41, 1813 (1963)) bekannt. Ihre Safener-Wirkungen waren jedoch nicht erkannt worden.

Gegenstand der vorliegenden Erfindung sind daher auch die bisher nicht beschriebenen Verbindungen der Formel I. Es sind dies die oben definierten Verbindungen der Formel I mit Ausnahme der Verbindungen, bei denen

a) $(Z)_n$ = H, (mono )$CH_3$, 4-$OCH_3$, 4-$NO_2$, 3,5-$(NO_2)_2$, 4-Br, 3-$CH_2$-OH oder 3-$CH_2$-O-$CH_2CH_2CH$-$(CH_3)_2$;
$(Z_1)_n$ = H und X = OH, $OCH_3$ oder $OCH_2$-$CH_3$ oder

b) $(Z)_n$ = H, $(Z_1)_n$ = 3,5- $(NO_2)_2$, 4-$CH_3$, 4-$OCH_3$, 4-$NO_2$, 4-Br oder 2-$NH_2$ und X = OH, $OCH_3$ oder $OCH_2$-$CH_3$ bedeuten.

Gegenstand der vorliegenden Erfindung ist auch ein Verfahren zur Herstellung der neuen Verbindungen der Formel I und deren Salze, dadurch gekennzeichnet, daß man eine Verbindung der Formel II

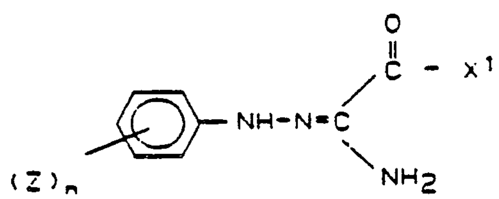

(II),

worin $X^1$ die Bedeutung von X außer Hydroxy besitzt,

a) mit einer Verbindung der Formel

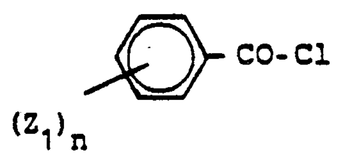

oder

b) mit einem Säureanhydride der Formel

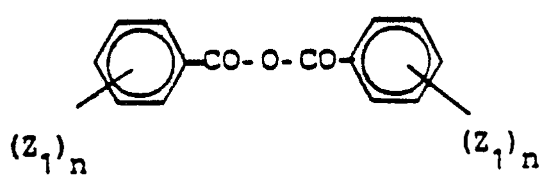

oder

c) mit einem Orthoester der Formel

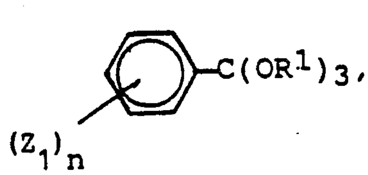

worin

$R^1$ = $(C_1$-$C_4)$Alkyl bedeutet,

umsetzt,

im Falle der Varianten a) und b) das Reaktionsgemisch gegebenenfalls anschließend in Essigsäure erhitzt und die erhaltenen Verbindungen der Formel I gegebenenfalls durch Derivatisierung in andere Verbindungen der Formel I oder deren Salze überführt.

Die Umsetzungen der Verbindungen der Formel II mit einem Carbonsäurechlorid, Säureanhydrid oder Orthoester werden zweckmäßigerweise in einem organischen inerten protonenfreien Lösungsmittel durchgeführt. Im Falle des Säureanhydrids oder Orthoesters (Variante b) oder c)) kann das betreffende Reagens auch selbst als Lösungsmittel dienen. Die Verfahrensvariante c) läßt sich vorteilhaft in Gegenwart eines sauren Katalysators, insbesondere einer organischen Säure wie p-Toluolsulfonsäure, durchführen.

Als inerte Lösungsmittel für die Verfahrensvarianten a), b) und c) eignen sich insbesondere Aromaten wie Benzol, Toluol, Xylol, Chlorbenzol oder cyclische Ether-Verbindungen wie Tetrahydrofuran oder Dioxan oder auch Ketone wie Aceton und dipolar aprotische Lösungsmittel wie Dimethylformamid. Die Reaktionstemperaturen variieren je nach Lösungsmittel zwischen 10°C und dem Siedepunkt des Reaktionsgemisches. Bei Einsatz von aromatischen Lösungsmitteln im Falle von Variante a) wird nach Zugabe des Carbonsäurechlorids das entstehende Wasser unter Rückfluß mittels eines Wasserabscheiders entfernt. Verschiedentlich entsteht im Falle der Verfahrensvarianten a) oder b) in Abhängigkeit der Rest $(Z)_n$, $(Z_1)_n$ und X der Verbindungen der Formel II zunächst ein Zwischenprodukt der Formel III,

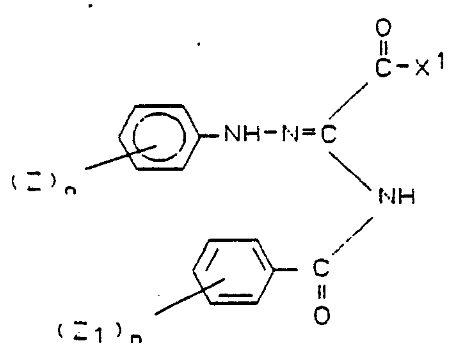

(III)

welches unter Umständen isoliert werden kann. Wenn die Reaktion auf dieser Stufe bei Verwendung der obengenannten Lösungsmittel stehen bleibt, muß eine Nachreaktion in Essigsäure durchgeführt werden. Hierzu wird das Zwischenprodukt der Formel III in Essigsäure zwischen ca. 50°C und Rückflußtemperatur erhitzt. Diese Nachreaktion kann im Eintopfverfahren durchgeführt werden, wobei vor Zugabe der Essigsäure das organische Lösungsmittel der ersten Verfahrensstufe abdestilliert wird.

Die so erhaltenen Verbindungen der Formel I können durch übliche Derivatisierungsreaktionen in andere Verbindungen der Formel I überführt werden. So können die Verbindungen der Formel I mit X = OH aus den Ester-Verbindungen der Formel I durch saure oder alkalische Hydrolyse gewonnen werden. Aus den Säuren der Formel I (X = OH) können auf üblichem Wege durch Zugabe entsprechender Basen die Salze der Verbindungen der Formel I erhalten werden. Ferner können aus den Esterverbindungen der Formel I in üblicher Weise andere Ester oder Amide der Formel I gewonnen werden, beispielsweise über die entsprechenden Säurechloride.

Die Herstellung von Verbindungen der Formel II ist im Prinzip bekannt; die Verbindungen in Formel II lassen sich durch Umsetzung von α-Chlorhydrazonen der Formel IV

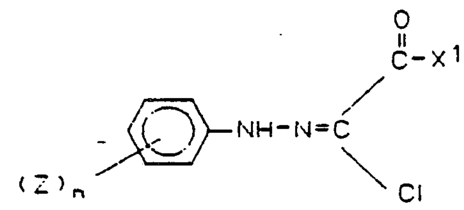

( I V )

mit Ammoniak herstellen. Die Verbindungen der Formel IV wiederum sind durch Umsetzung von Phenyldiazoniumsalzen mit α-Halogenacetessigestern bzw. α-Halogen-β-diketonen zugänglich. Beide Reaktionen sind in J. Chem. Soc. 87 1859 (1905) und Ber. d. dt. Chem. Ges. 50 1482 (1917) beschrieben.

Die Verbindungen der Formel I reduzieren oder unterbinden phytotoxische Nebenwirkungen von Pflanzenschutzmitteln, insbesondere von Herbiziden, die bei deren Einsatz in Nutzpflanzenkulturen auftreten können.

Die Verbindungen der Formel I können nacheinander oder zusammen mit den herbiziden Wirkstoffen ausgebracht werden. Sie sind dann in der Lage, schädliche Nebenwirkungen dieser Herbizide bei Kulturpflanzen zu vermindern oder völlig aufzuheben, ohne die Wirksamkeit dieser Herbizide gegen Schadpflanzen zu beeinträchtigen.

Hierdurch kann das Einsatzgebiet herkömmlicher Pflanzenschutzmittel ganz erheblich erweitert werden. Solche Verbindungen, die die Eigenschaft besitzen, Kulturpflanzen gegen phytotoxische Schäden durch Herbizide zu schützen, werden "Antidote" oder "Safener" genannt.

Herbizide, deren phytotoxische Nebenwirkungen mittels der Verbindungen der Formel I herabgesetzt werden können, sind z.B. Carbamate, Thiocarbamate, Halogenacetanilide, substituierte Phenoxy-, Naphthoxy- und Phenoxyphenoxycarbonsäurederivate sowie Heteroaryloxyphenoxycarbonsäurederivate wie Chinolyloxy-, Chinoxalyloxy-, Pyridyloxy-, Benzoxazolyloxy-, Benzthiazolyloxy-phenoxy-carbonsäureester und ferner Dimedonoximabkömmlinge. Bevorzugt hiervon sind Phenoxyphenoxy- und Heteroaryloxyphenoxycarbonsäureester. Als Ester kommen hierbei insbesondere niedere Alkyl-, Alkenyl und Alkinylester in Frage.

Beispielsweise seien, ohne daß dadurch eine Beschränkung erfolgen soll, folgende Herbizide genannt:

A) Herbizide vom Typ der Phenoxyphenoxy- und Heteroaryloxyphenoxycarbonsäure- ($C_1$-$C_4$)alkyl-, ($C_2$-$C_4$)alkenyl- und ($C_3$-$C_4$)alkinylester wie
2-(4-(2,4-Dichlorphenoxy)-phenoxy)-propionsäuremethylester,
2-(4-(4-Brom-2-chlorphenoxy)-phenoxy)-propionsäuremethylester,
2-(4-(4-Trifluormethylphenoxy)-phenoxy)-propionsäuremethylester,
2-(4-(2-Chlor-4-trifluormethylphenoxy)-phenoxy)-propionsäuremethylester,
2-(4-(2,4-Dichlorbenzyl)-phenoxy)-propionsäuremethylester, 4-(4-(4-Trifluormethylphenoxy)-phenoxy)-pont-2-ensäureethylester,
2-(4-(3,5-Dichlorpyridyl-2-oxy)-phenoxy)-propionsäureethylester,
2-(4-(3,5-Dichlorpyridyl-2-oxy)-phenoxy)-propionsäurepropargylester,
2-(4-(6-Chlorbenzoxazol-2-yl-oxy)-phenoxy)-propionsäureethylester,
2-(4-(3-Chlor-5-trifluormethyl-2-pyridyloxy)-phenoxy)-propionsäuremethylester,
2-(4-(5-Trifluormethyl-2-pyridyloxy)-phenoxy)-propionsäurebutylester,
2-(4-(6-Chlor-2-chinoxalyloxy)-phenoxy)-propionsäuremethylester,
2-(4-(6-Fluor-2-chinoxalyloxy)-phenoxy)-propionsäuremethylester,
2-(4-(6-Chlor-2-chinolyloxy)-phenoxy)-propionsäuremethylester,

B) Chloracetanilid-Herbizide wie
N-Methoxymethyl-2,6-diethyl-chloracetanilid,
N-(3′-Methoxyprop-2-yl)-methyl-6-ethyl-chloracetanilid,
N-(3-Methyl-1,2,4-oxdiazol-5-yl-methyl)-chloressigsäure-2,6-dimethylanilid,

C) Thiocarbamate wie
S-Ethyl-N,N-dipropylthiocarbamat oder
S-Ethyl-N,N-diisobutylthiooarbamat

D) Dimedon-Derivate wie
Methyl-3-(1-allyloxyimino)butyl)-4-hydroxy-6,6-dimethyl-2-oxocyclohex-3-encarboxylat
2-(N-Ethoxybutyrimidoyl)-5-(2-ethylthiopropyl)-3-hydroxy-2-cyclohexen-1-on,
2-(N-Ethoxybutyrimidoyl)-5-(2-phenylthiopropyl)-3-hydroxy-2-cyclohexen-1-on,
2-(1-Allyloxyiminobutyl)-4-methoxycarbonyl-5,5-dimethyl-3-oxocyclohexenol,
2-(1-(3-Chlorallyloxy)iminobutyl)-5-(2-ethylthio)propyl)-3-hydroxy-cyclohex-2-enon

2-(1-(Ethoxyimino)-butyl)-3-hydroxy-5-(thian-3-yl)-cyclohex-2-enon oder
2-(1-Ethoxyiminopropyl)-5-(2,4,6-trimethylphenyl)-3-hydroxy-2-cyclohexen-1-on.

Das Mengenverhältnis Safener : Herbizid kann innerhalb weiter Grenzen, im Bereich zwischen 1 : 10 und 10 : 1, insbesondere zwischen 2 : 1 und 1 : 10, schwanken. Die jeweils optimalen Mengen an Herbizid und Safener sind abhängig vom Typ des verwendeten Herbizids oder vom verwendeten Safener sowie von der Art des zu behandelnden Pflanzenbestandes und lassen sich von Fall zu Fall durch entsprechende Versuche ermitteln.

Haupteinsatz für die Anwendung der Safener sind vor allem Getreidekulturen (Weizen, Roggen, Gerste, Hafer), Reis, Mais, Sorghum, aber auch Baumwolle, Zuckerrübe, Zuckerrohr und Sojabohne.

Die Safener der Formel I können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläohe vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Verbindungen der Formel I können je nach Indikation und verwendetem Herbizid innerhalb weiter Grenzen schwanken und variieren im allgemeinen zwischen 0,01 und 10 kg Wirkstoff je Hektar.

Gegenstand der vorliegenden Erfindung ist auch ein Verfahren zum Schutz von Kulturpflanzen vor phytotoxischen Nebenwirkungen von Herbiziden, das dadurch gekennzeichnet ist, daß eine wirksame Menge einer Verbindung der Formel I vor, nach oder gleichzeitig mit dem Herbizid appliziert wird.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, Emulsionen, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder duch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

Die erfindungsgemäßen Mittel mit einem Safener als alleiniger Aktivsubstanz oder mit einem Safener und einem Herbizid als Aktivsubstanzen enthalten in der Regel 2 bis 95 Gew.-% Aktivsubstanz.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Diese oben genannten Formulierungstypen werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die für diese Formulierungen zu verwendenden Formulierungshilfsmittel (Inertmaterialien, Emulgatoren, Netzmittel, Tenside, Lösungsmittel etc.) sind beispielsweise in Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood oder "Encyclopedia of Surface Active Agents", Chem. Publ. Co. Inc., N.Y. 1964 beschrieben.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

**Formulierungsbeispiele**

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether (8 EO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 EO) als Emulgator.

E. Ein in Wasser leicht emulgierbares Konzentrat aus einem Phenoxycarbonsäureester und einem Safener (10 : 1) wird erhalten aus

12,00 Gew.-% 2-[4-(4-Chlorbenzoxazol-2-yl-oxy)-phenoxy]-propionsäureethylester
1,20 Gew.-% Verbindung der Formel I
69,00 Gew.-% Xylol
7,80 Gew.-% dodecylbenzolsulfonsaurem Calcium
6,00 Gew.-% ethoxyliertem Nonylphenol (10 EO)
4,00 Gew.-% ethoxyliertem Rizinusöl (40 EO)

Die Zubereitung erfolgt wie unter Beispiel A) angegeben.

F. Ein in Wasser leicht emulgierbares Konzentrat aus einem Phenoxycarbonsäureester und einem Safener (1 : 10) wird erhalten aus

4,0 Gew.-% 2-[4-(6-Chlorbenzoxazol-2-yl-oxy)-phenoxy]-propionsäureethylester
40,0 Gew.-% Verbindung der Formel I
30,0 Gew.-% Xylol
20,0 Gew.-% Cyclohexanon

4,0 Gew.-% dodecylbenzolsulfonsaurem Calcium
2,0 Gew.-% ethoxyliertem Rizinusöl (40 EO)

## Chemische Beispiele

### 1) 1-(2,4-Dichlorphenyl)-3-ethoxy-carbonyl-5-phenyl-1,2,4-triazol.

a) in Toluol als Lösungsmittel

26,1 g (0,1 Mol) α-Amino-α-(2,4-Dichlor-phenylhydrazono)-glyoxylsäuremethylester wurden in 150 ml Toluol vorgelegt, 0,12 Mol Benzoesäureanhydrid unter Rühren portionsweise fest zugegeben und anschließend unter Rückfluß so lange am Wasserabscheider gekocht, bis kein Wasser mehr übergeht (ca. 1 Std.).

Nach Abkühlen wurde die Toluollösung mit Wasser gewaschen und das Toluol im Vakuum abdestilliert. Das zurückbleibende Rohprodukt wurde aus Cyclohexan umkristallisiert.
Ausbeute: 27, 15 g = 75 %
Weiße Kristalle vom Schmp. 113-115 °C.

### 2. 1-(2,4-Dichlorphenyl)-5-phenyl-1,2,4-triazol-3-carbonsäure

124,3 g (0,343 Mol) 1-(2,4-Dichlorphenyl)-3-ethoxycarbonyl-5-phenyl-1,2,4-triazol wurden in 1500 ml Methanol vorgelegt und mit 0,343 Mol NaOH (13, 7 g) in 80 ml $H_2O$ versetzt. Nach 5-stündigem Rühren bei Raumtemperatur wurde auf 5,5 l Wasser gegossen, von unlöslicher Substanz filtriert und das klare Filtrat mit Salzsäure auf pH 1 gestellt. Die ausfallenden Kristalle wurden abgesaugt, mit $H_2O$ gewaschen und getrocknet.

| | |
|---|---|
| Ausbeute: | 114,56 g = 99,9 % |
| Schmelzpunkt: | 180-182 °C. |

### 3. 1-(2,4-Dichlorphenyl)-5-phenyl-3-isopropoxy-carbonyl-1,2,4-triazol

33, 4 g (0, 1 Mol) 1- (2,4-Dichlorphenyl)-5-phenyl-1,2,4-triazol-3-carbonsäure wurden in 37 ml Thionylchlorid 30 Minuten unter Rückfluß erhitzt, das überschüssige Thionylchlorid im Vakuum entfernt und das rohe Carbonsäurechlorid in 200 ml Isopropanol 60 Minuten gekocht. Nach Erkalten der Lösung wurde in Eiswasser gegossen; die Kristalle wurden abgesaugt und an der Luft getrocknet.
Ausbeute: 18,8 g (49,9 %)
Schmelzpunkt: 145 °C.

### 4. 1-(2,4-Dichlorphenyl)-5-phenyl-1,2,4-triazol-3-carbonsäureanilid

33, 4 g (0,1 Mol) 1-(2,4-Dichlorphenyl)-5-phenyl-1,2,4-triazol-3-carbonsäure wurde analog Beispiel 3 in das Säurechlorid überführt, dieses in 240 ml Toluol gelöst und bei +5 °C in 20 Minuten tropfenweise mit einer Mischung aus 0,1 Mol Anilin und 0,1 mol Triethylamin versetzt. Nach 5 stündigem Rühren bei Raumtemperatur wurde mit Wasser gewaschen und das Toluol im Vakuum entfernt. Der Rückstand wurde mit $H_2O$ gewaschen und an der Luft getrocknet.
Ausbeute: 16,3 g (39,8 %)
Schmelzpunkt: 190 °C

Analog den Herstellungsbeispielen 1-4 wurden die in Tabelle 1 aufgeführten Verbindungen der Formel I hergestellt.

**Tabelle 1**

| Bsp. Nr. | $(Z)_n$ | $(Z_1)_n$ | X | Schmp. (°C) |
|---|---|---|---|---|
| 5 | 2,4-$Cl_2$ | H | $-OCH_3$ | 183 |
| 6 | " | " | $-OCH_2CH_2CH_3$ | 150 |
| 7 | " | " | $-OCH_2CH_2CH_2CH_3$ | 135 |
| 8 | " | " | $-OCH_2-CH(CH_3)_2$ | 145 |
| 9 | " | " | $-OCH_2CO_2CH_2CH_3$ | 139 |
| 10 | " | " | $-O-\underset{CH_3}{\underset{\vert}{CH}}-CO_2CH_2CH_3$ | Öl |
| 11 | " | " | $-OCH_2CO_2C_3H_7$ | |
| 12 | " | " | $-OCH_2CO_2CH_3$ | 124 |
| 13 | " | " | $-O\underset{CH_3}{\underset{\vert}{CH}}CO_2CH_3$ | 111 |
| 14 | " | " | $-NH_2$ | 215 |
| 15 | " | " | $-NHC_6H_4-4-Cl$ | |
| 16 | " | " | $-NH-NH_2$ | 216 |

**Tabelle 1 (Fortsetzung)**

| Bsp. Nr. | $(Z)_n$ | $(Z_1)_n$ | X | Schmp. (°C) |
|---|---|---|---|---|
| 17 | 2,4-$Cl_2$ | H | $-N(CH_3)_2$ | |
| 18 | " | " | 2,6-dimethyl-morpholin-4-yl | |
| 19 | " | " | $-NHC_6H_4-3-CF_3$ | |
| 20 | 2-Cl | " | $-OCH_2CH_3$ | |
| 21 | " | " | $-OCH_3$ | |
| 22 | " | " | $-OCH_2CH_2CH_3$ | |
| 23 | " | " | $-OCH_2CH_2CH_2CH_3$ | |
| 24 | " | " | $-OCH_2-CH(CH_3)_2$ | |
| 25 | " | " | $-OH$ | |
| 26 | " | " | $-OCH_2CO_2CH_2CH_3$ | |
| 27 | " | " | $-O-\underset{CH_3}{CH}-CO_2CH_2CH_3$ | |
| 28 | " | " | $-OCH_2CO_2C_2H_5$ | |
| 29 | " | " | $-OCH_2CO_2CH_3$ | |
| 30 | " | " | $-O\underset{CH_3}{C}HCO_2CH_3$ | |
| 31 | " | " | $-NH_2$ | |
| 32 | " | " | $-NHC_6H_4-4-Cl$ | |
| 33 | " | " | $-NH-NH_2$ | |
| 34 | " | " | $-N(CH_3)_2$ | |
| 35 | " | " | 2,6-dimethyl-morpholin-4-yl | |
| 36 | " | " | $-NHC_6H_4-3-CF_3$ | |
| 37 | 4-Cl | " | $-OCH_2CH_3$ | |
| 38 | " | " | $-OCH_3$ | |
| 39 | " | " | $-OCH_2CH_2CH_3$ | |
| 40 | " | " | $-OCH_2CH_2CH_2CH_3$ | |
| 41 | " | " | $-OCH_2CH(CH_3)_2$ | |
| 42 | " | " | $-OH$ | |
| 43 | " | " | $-OCH_2CO_2CH_2CH_3$ | |

Tabelle 1 (Fortsetzung)

| Bsp. Nr. | $(Z)_n$ | $(Z_1)_n$ | X | Schmp. (°C) |
|---|---|---|---|---|
| 44 | 4-Cl | H | $-O-CH-CO_2CH_2CH_3$<br>$CH_3$ | |
| 45 | " | " | $-OCH_2CO_2C_2H_5$ | |
| 46 | " | " | $-OCH_2CO_2CH_3$ | |
| 47 | " | " | $-OCHCO_2CH_3$<br>$CH_3$ | |
| 48 | " | " | $-NH_2$ | |
| 49 | " | " | $-NHC_6H_4Cl(p)$ | |
| 50 | " | " | $-NH-NH_2$ | |
| 51 | " | " | $-N(CH_3)_2$ | |
| 52 | " | " | 2,6-dimethyl-morpholin-4-yl | |
| 53 | " | " | $-NHC_6H_4CF_3(m)$ | |
| 54 | $2,4-Cl_2$ | " | -O-CH-C≡CH | |
| 55 | " | 2-Cl | " | |
| 56 | " | 4-Cl | " | |
| 57 | " | $2,4-Cl_2$ | " | |
| 58 | " | H | $-O-CH_2-CH=CH_2$ | |
| 59 | " | 2-Cl | " | |
| 60 | " | 4-Cl | " | |
| 61 | " | $2,4-Cl_2$ | " | |
| 62 | $2,4-Cl_2$ | 4-Cl | OH | |
| 63 | " | " | $-OCH_2CH_3$ | 117 |
| 64 | " | " | $-OCH_3$ | |
| 65 | " | " | $-OCH_2CH_2CH_3$ | |
| 66 | " | " | $-OCH(CH_3)_2$ | |
| 67 | " | " | $-O-CH_2CO_2C_2H_5$ | |
| 68 | $2,4-Cl_2$ | 2-Cl | -OH | |
| 69 | " | " | $-OCH_2CH_3$ | |
| 70 | " | " | $-OCH_3$ | |
| 71 | " | " | $-O-CH_2-CO_2C_2H_5$ | |

Tabelle 1 (Fortsetzung)

| Bsp. Nr. | $(Z)_n$ | $(Z_1)_n$ | X | Schmp. (°C) |
|---|---|---|---|---|
| 72 | 2-Cl | 4-Cl | $-OCH_2CH_3$ | |
| 73 | " | " | -OH | |
| 74 | " | " | $-OCH_2CO_2C_2H_5$ | |
| 75 | $2,4-Cl_2$ | H | $-O^-Na^+$ | 190-200 |
| 76 | " | " | $-O^-K^+$ | 200-210 |
| 77 | " | " | $-O^-NH_4^+$ | 190-200 |
| 78 | " | " | $-O-CH(CH_3)_2$ | 132 |
| 79 | " | $2,4-Cl_2$ | $OCH_3$ | 145 |
| 80 | " | " | $OC_2H_5$ | 150 |
| 81 | " | " | OH | 194 |
| 82 | " | " | $OCH_2CH_2CH_3$ | |
| 83 | " | " | $OCH(CH_3)_2$ | 159 |
| 84 | " | " | $OCH_2CH_2CH_2CH_3$ | |
| 85 | " | " | $OCH_2CO_2CH_3$ | |
| 86 | " | " | $OCH_2CO_2C_2H_5$ | |
| 87 | " | " | $OCH_2CH(CH_3)_2$ | |
| 88 | " | " | $O^-Na^+$ | |
| 89 | " | " | $O^-K^+$ | |
| 90 | " | " | $O^-NH_4^+$ | |
| 91 | " | " | $NHNH_2$ | |
| 92 | " | " | $OCH(CH_3)-CO_2C_2H_5$ | |
| 93 | " | " | $OCH(CH_3)-CO_2CH_3$ | |
| 94 | " | " | $NH_2$ | |
| 95 | " | " | $N(CH_3)_2$ | |
| 96 | H | H | $OCH_3$ | |
| 97 | " | " | $OC_2H_5$ | 171 |
| 98 | $2,4-Cl_2$ | $2,4-Cl_2$ | $OCH(C_2H_5)-CONHCH_2C_6H_5$ | |
| 99 | " | " | $OCH(C_2H_5)-CONHC_6H_5$ | |

**Biologische Beispiele**

**Beispiel 1**

Weizen und Gerste wurden im Gewächshaus in Plastiktöpfen bis zum 3-4 Blattstadium herangezogen und dann nacheinander mit den erfindungsgemäßen Verbindungen und den getesteten Herbiziden im Nachauflaufverfahren behandelt. Die Herbizide und die Verbindungen der Formel I wurden dabei in Form wäßriger Suspensionen bzw. Emulsionen mit einer Wasseraufwandmenge von umgerechnet 800 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde.

Die Ergebnisse aus Tabelle 2 veranschaulichen, daß die erfindungsgemäßen Verbindungen starke Herbizidschäden an den Kulturpflanzen effektiv reduzieren können.

Selbst bei starken Überdosierungen des Herbizids H werden bei den Kulturpflanzen auftretende schwere Schädigungen deutlich reduziert, geringere Schäden völlig aufgehoben. Mischungen aus Herbiziden und erfindungsgemäßen Verbindungen eignen sich deshalb in ausgezeichneter Weise zur selektiven Unkrautbekämpfung in Getreidekulturen.

Tabelle 2

| Safenerwirkung der erfindungsgemäßen Verbindungen | | | | |
|---|---|---|---|---|
| | Bsp. Nr. | Dosis kg a.i./ha | Kulturpflanzenschädigung | |
| | | | TRAE | HOVU |
| H | | 2,0 | 75 | - |
| | | 0,2 | - | 80 |
| H + | 3 | 2,0 + 2,5 | 40 | - |
| | | 0,2 + 2,5 | - | 35 |
| H + | 4 | 2,0 + 2,5 | 40 | - |
| | | 0,2 + 2,5 | - | 45 |
| H + | 7 | 2,0 + 2,5 | 35 | - |
| | | 0,2 + 2,5 | - | 40 |
| H + | 8 | 2,0 + 2,5 | 50 | - |
| | | 0,2 + 2,5 | - | 40 |
| Abkürzungen: | | | | |

TRAE = Triticum aestivum
HOVU = Hordeum vulgare
a.i. = Aktivsubstanz
H = 2-(4-(6-Chlorbenzoxazol-2-yloxy)phenoxypropionsäureethylester

**Ansprüche**

1. Mittel zur Verwendung als Safener für Herbizide, dadurch gekennzeichnet, daß sie eine Verbindung der Formel I oder deren Salz,

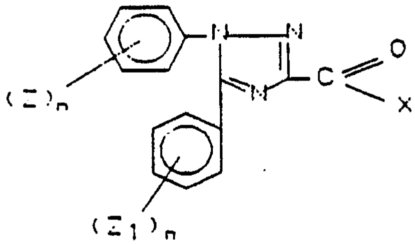

(I)

worin

Z und $Z_1$ unabhängig voneinander Halogen, Nitro, Cyano, ($C_1$-$C_4$)-Alkyl, ($C_1$-$C_4$)Alkoxy, ($C_1$-$C_4$)-Alkylthio, wobei die Alkyl-, Alkoxy- und Alkylthiogruppen durch ein oder mehrere Halogenatome, insbesondere Fluor oder Chlor, substituiert sein können, ($C_3$-$C_6$)Cycloalkyl, das durch ($C_1$-$C_4$)Alkyl substituiert sein kann, Amino, ($C_1$-$C_4$)Alkylamino, ($C_1$-$C_4$)-Dialkylamino, Hydroxymethyl, ($C_1$-$C_4$)Alkoxymethyl, wobei die Alkyl- und Alkoxygruppen in den vorgenannten Resten durch ($C_1$-$C_4$)Alkyl substituiert sein können, Phenyl oder Phenoxy, wobei Phenyl und Phenoxy durch Halogen ein- oder mehrfach und/oder einfach durch Trifluormethyl substituiert sein können.

X Hydroxy, $-OCH_2Si(CH_3)_3$, ($C_1$-$C_4$)Alkyl, ($C_3$-$C_6$)Cycloalkoxy, Phenyl($C_1$-$C_6$)alkoxy, Phenoxy, ($C_2$-$C_6$)-Alkenyloxy, ($C_2$-$C_6$)-Alkinyloxy, ($C_1$-$C_6$)Alkoxy, ($C_1$-$C_6$)Alkylthio, wobei die Alkoxy oder Alkylthiogruppe ein- oder zweifach durch ($C_1$-$C_2$)Alkoxy, Mono- oder Di- ($C_1$-$C_4$)Alkyl-phenylaminocarbonyl, Mono- oder Di- ($C_1$-$C_6$)Alkylamino, ($C_1$-$C_6$)Alkyl-carbonyloxy, ($C_1$-$C_2$)Alkylthio, Cyano oder ein- oder mehrfach durch Halogen substituiert sein kann, einen Rest der Formeln

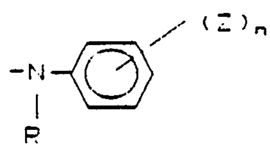

oder

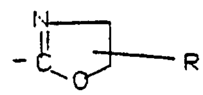

worin jeweils R Wasserstoff oder ($C_1$-$C_4$)Alkyl bedeutet, ferner Mono- oder Di- ($C_1$-$C_4$)Alkylamino, ($C_5$-$C_6$)-Cycloalkylamino, Piperidino, Morpholino oder 2,6-Dimethylmorpholino, einen Rest der Formel

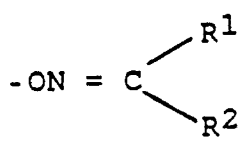

worin $R^1$ und $R^2$ unabhängig voneinander ($C_1$-$C_4$)Alkyl bedeuten, oder $R^1$ und $R^2$ gemeinsam mit dem sie verknüpfenden C-Atom einen 5-, 6- oder 7-gliedrigen Cycloalkylrest bilden können,
ferner einen Rest der Formel

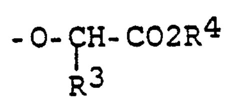

worin $R^3$ und $R^4$ unabhängig voneinander H oder ($C_1$-$C_4$)Alkyl bedeuten, und
n die Zahl 0, 1, 2 oder 3 bedeuten, und inerte Trägerstoffe enthalten.

2. Mittel gemäß Anspruch 1, dadurch gekennzeichnet, daß
Z, $Z_1$ = Chlor, Fluor, ($C_1$-$C_4$)Alkoxy, ($C_1$-$C_4$)Alkyl oder $CF_3$,
X = ($C_1$-$C_6$)Alkoxy oder Hydroxy und
n = 0, 1, 2 oder 3 bedeuten.

3. Mittel gemäß Anspruch 1 oder 2, dadurch gekennzeichnet, daß sie zusätzlich ein Herbizid aus der Gruppe der Thiocarbamate, Carbamate, Halogenacetanilide, Phenoxy-, Naphthoxy-, Phenoxyphenoxy- oder Heteroaryloxyphenoxycarbonsäureester oder der Dimedonoximderivate enthalten.

4. Mittel gemäß Anspruch 3, dadurch gekennzeichnet, daß man als Herbizid ein Phenoxyphenoxy- oder

Heteroaryloxyphenoxy-carbonsäureester einsetzt.

5. Verfahren zum Schutz von Kulturpflanzen gegen phytotoxische Nebenwirkungen von Herbiziden, dadurch gekennzeichnet, daß man die Pflanzen, Pflanzensamen oder Anbauflächen mit einer wirksamen Menge einer Verbindung der Formel I von Anspruch 1 oder 2 vor, nach oder gleichzeitig mit dem Herbizid behandelt.

6. Verwendung von Verbindungen der Formel I von Anspruch 1 oder 2 zum Schutz von Kulturpflanzen gegen phytotoxische Nebenwirkungen von Herbiziden.

7. Verbindungen der Formel I von Anspruch 1 mit Ausnahme der Verbindungen, worin

a) $(Z)_n$ = H, $CH_3$, 4-$OCH_3$, 4-$NO_2$, 3,5-$(NO_2)_2$, 4-Br, 3-$CH_2$-OH oder 3-$CH_2$-O-$CH_2CH_2CH(CH_3)_2$; $(Z_1)n$ = H und X = OH, $OCH_3$ oder $OCH_2$-$CH_3$ oder

b) $(Z)_n$ = H, $(Z_1)_n$ = 3,5-$(NO_2)_2$, 4-$CH_3$, 4-$OCH_3$, 4-$NO_2$, 4-Br oder 2-$NH_2$ und X = OH, $OCH_3$ oder $OCH_2$-$CH_3$ bedeuten.

8. Verfahren zur Herstellung der Verbindungen der Formel I von Anspruch 7, dadurch gekennzeichnet, daß man eine Verbindung der Formel II

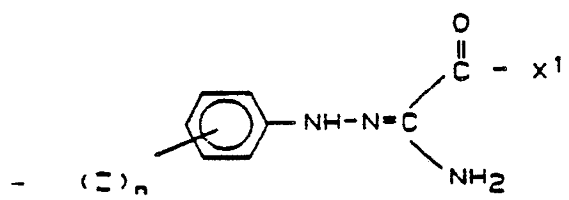

(II),

worin $X^1$ die Bedeutung von X außer Hydroxy besitzt,

a) mit einer Verbindung der Formel

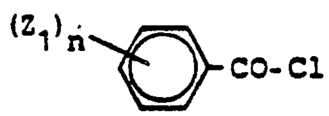

oder

b) mit einem Säureanhydrid der Formel

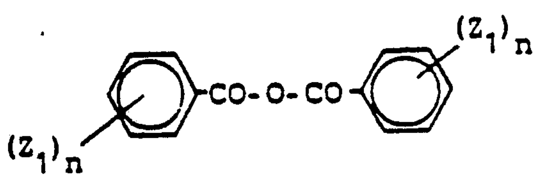

oder

c) mit einem Orthoester der Formel

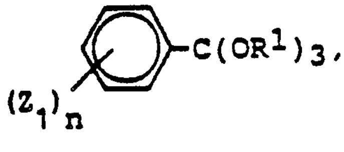

worin

$R^1$ = $(C_1$-$C_4)$Alkyl bedeutet,

umsetzt,

im Falle der Varianten a) und b) das Reaktionsgemisch gegebenenfalls anschließend in Essigsüure erhitzt und die erhaltenen Verbindungen der Formel I gegebenenfalls durch Derivatisierung in andere Verbindungen der Formel I oder deren Salze überführt.

Patentansprüche für folgenden Vertragsstaat: ES

1. Verwendung einer Verbindung der Formel I

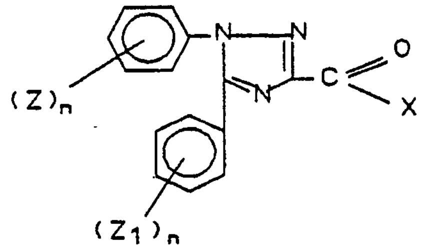

(I)

worin

Z und $Z_1$ unabhängig voneinander Halogen, Nitro, Cyano, $(C_1$-$C_4)$-Alkyl, $(C_1$-$C_4)$Alkoxy, $(C_1$-$C_4)$-Alkylthio, wobei die Alkyl-, Alkoxy- und Alkylthiogruppen durch ein oder mehrere Halogenatome, insbesondere Fluor oder Chlor, substituiert sein können, $(C_3$-$C_6)$Cycloalkyl, das durch $(C_1$-$C_4)$Alkyl substituiert sein kann, Amino, $(C_1$-$C_4)$Alkylamino, $(C_1$-$C_4)$-Dialkylamino, Hydroxymethyl, $(C_1$-$C_4)$Alkoxymethyl, wobei die Alkyl- und Alkoxygruppen in den vorgenannten Resten durch $(C_1$-$C_4)$Alkyl substituiert sein können, Phenyl oder Phenoxy, wobei Phenyl und Phenoxy durch Halogen ein- oder mehrfach und/oder einfach durch Trifluormethyl substituiert sein können,

X Hydroxy, $-OCH_2Si(CH_3)_3$, $(C_1$-$C_4)$Alkyl, $(C_3$-$C_6)$Cycloalkoxy, Phenyl$(C_1$-$C_6)$alkoxy, Phenoxy, $(C_2$-$C_6)$-Alkenyloxy, $(\Psi_2$-$C_6)$-Alkinyloxy, $(C_1$-$C_6)$Alkoxy, $(C_1$-$C_6)$Alkylthio, wobei die Alkoxy oder Alkylthiogruppe ein- oder zweifach durch $(C_1$-$C_2)$Alkoxy, Mono- oder Di- $(C_1$-$C_4)$Alkyl-phenylaminocarbonyl, Mono- oder Di- $(C_1$-$C_6)$Alkylamino, $(C_1 C_6)$Alkyl-carbonyloxy, $(C_1$-$C_2)$Alkylthio, Cyano oder ein- oder mehrfach durch Halogen substituiert sein kann, einen Rest der Formeln

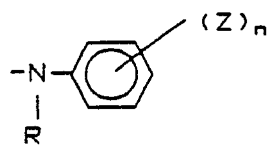

oder

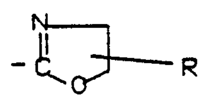

worin jeweils R Wasserstoff oder $(C_1$-$C_4)$Alkyl bedeutet, ferner Mono- oder Di- $(C_1$-$C_4)$Alkylamino, $(C_5$-$C_6)$-Cycloalkylamino, Piperidino, Morpholino oder 2,6-Dimethylmorpholino, einen Rest der Formel

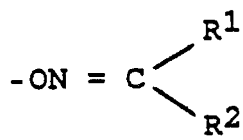

worin $R^1$ und $R^2$ unabhängig voneinander $(C_1$-$C_4)$Alkyl bedeuten, oder $R^1$ und $R^2$ gemeinsam mit dem sie verknüpfenden C-Atom einen 5-, 6- oder 7-gliedrigen Cycloalkylrest bilden können,
ferner einen Rest der Formel

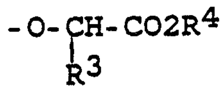

worin $R^3$ und $R^4$ unabhängig voneinander H oder $(C_1$-$C_4)$Alkyl, bedeuten, und

n die Zahl 0, 1, 2 oder 3 bedeuten,

als Safener für Herbizide.

2. Verwendung gemäß Anspruch 1, dadurch gekennzeichnet, daß

$Z, Z_1$ = Chlor, Fluor, $(C_1-C_4)$Alkoxy, $(C_1-C_4)$Alkyl oder $CF_3$,
X = $(C_1-C_6)$Alkoxy oder Hydroxy und
n = 0, 1, 2 oder 3 bedeuten.

3. Verwendung gemäß Ansprüchen 1 oder 2, dadurch gekennzeichnet, daß als Herbizid eines aus der Gruppe der Thiolcarbamate, Carbamate, Halogenacetanilide, Phenoxy-, Naphthoxy-, Phenoxyphenoxy- oder Heteroaryloxyphenoxycarbonsäureester oder der Dimedonoximderivate eingesetzt wird.

4. Verwendung gemäß Anspruch 3, dadurch gekennzeichnet, daß man als Herbizid ein Phenoxyphenoxy- oder Heteroaryloxyphenoxy-carbonsäureester einsetzt.

5. Verwendung nach einem oder mehreren der Ansprüche 1 bis 4, dadurch gekennzeichnet, daß Verbindungen der Formel I mit Ausnahme der Verbindungen eingesetzt werden, worin

a) $(Z)_n$ = H, $CH_3$, 4-$OCH_3$, 4-$NO_2$, 3,5-$(NO_2)_2$, 4-Br, 3-$CH_2$-OH oder 3-$CH_2$-O-$CH_2CH_2CH(CH_3)_2$; $(Z_1)_n$ = H und X = OH, $OCH_3$ oder $OCH_2$-$CH_3$ oder

b) $(Z)_n$ = H, $(Z_1)_n$ = 3,5-$(NO_2)_2$, 4-$CH_3$, 4-$OCH_3$, 4-$NO_2$, 4-Br oder 2-$NH_2$ und X = OH, $OCH_3$ oder $OCH_2$-$CH_3$ bedeuten.

6. Verfahren zur Herstellung der Verbindungen der Formel I von Anspruch 5, dadurch gekennzeichnet, daß man eine Verbindung der Formel II

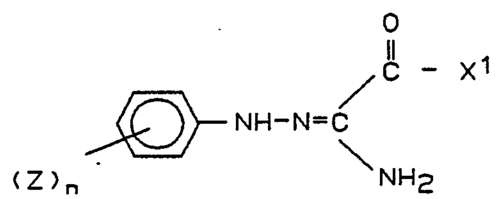

(II),

worin $X^1$ die Bedeutung von X außer Hydroxy besitzt,

a) mit einer Verbindung der Formel 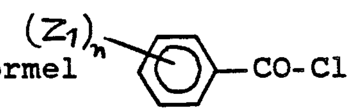 oder

b) mit einem Säureanhydrid der Formel 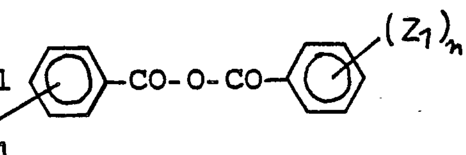

c) mit einem Orthoester der Formel 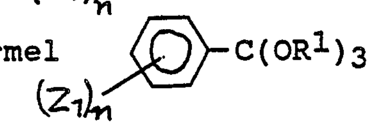, worin

$R^1$ = $(C_1-C_4)$Alkyl bedeutet,
umsetzt,
im Falle der Varianten a) und b) das Reaktionsgemisch gegebenenfalls anschließend in Essigsüure erhitzt wird,
und die erhaltenen Verbindungen der Formel I gegebenenfalls durch Derivatisierung in andere Verbindungen der Formel I oder deren Salze überführt.

7. Verfahren zum Schutz von Kulturpflanzen gegen phytotoxische Nebenwirkungen von Herbiziden, dadurch gekennzeichnet, daß man die Pflanzen, Pflanzensamen oder Anbauflächen mit einer wirksamen Menge einer Verbindung der Formel I von Anspruch 1 oder 2 vor, nach oder gleichzeitig mit dem Herbizid behandelt.

Europäisches Patentamt

# EUROPÄISCHER RECHERCHENBERICHT

Nummer der Anmeldung

EP 89 10 8715

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 86, Nr. 11, 14. März 1977, Seite 605, Zusammenfassung Nr. 72526z, Columbus, Ohio, US; M. RUCCIA et al.: "A 1,3-dipolar cycloaddition reaction of 1-methylindazole", & HETEROCYCLES 1976, 4(10), 1655-8<br>* Zusammenfassung * | 7,8 | C 07 D 249/10<br>A 01 N 25/32 |
| X | CHEMICAL ABSTRACTS, Band 90, Nr. 9, 26. Februar 1979, Seite 510, Zusammenfassung Nr. 72117z, Columbus, Ohio, US; M. RUCCIA et al.: "Addition products of indazole and nitrilimines. Synthesis of 1-phenyl-5-(o-aminophenyl)-1,2,4-triazoles and their annelation to 1,2,4-triazolo[1,5-f]phenanthridines", & HETEROCYCLES 1978, 9(11), 1577-83<br>* Zusammenfassung * | 7,8 | |
| X | FR-A-2 526 271 (KUREHA K.K.K.K.)<br>* Insgesamt * | 1-4,7,8 | **RECHERCHIERTE SACHGEBIETE (Int. Cl.4)** |
| X | AUSTRALIAN JOURNAL OF CHEMISTRY, Nr. 27, 1974, Seiten 2509-2510, East Melbourne, AU; A.M. KHALIL et al.: "Reaction of 2-aryl-4-arylazo-2-oxazolin-5-ones with amines"<br>* Insgesamt * | 7,8 | C 07 D 249/00<br>A 01 N 25/00 |

-/-

Der vorliegende Recherchenbericht wurde für alle Patentansprüche erstellt

| Recherchenort | Abschlußdatum der Recherche | Prüfer |
|---|---|---|
| DEN HAAG | 08-08-1989 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung
EP 89 10 8715

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | AUSTRALIAN JOURNAL OF CHEMISTRY, Nr. 29, 1976, Seiten 1627-1629, East Melbourne, AU; A.M. KHALIL et al.: "Synthesis of 2-aryl-4-arylazo-2-thiazolin-5-ones and their reaction with aromatic amines" * Insgesamt * --- | 7,8 | |
| X | BULLETIN DE LA SOCIETE CHIMIQUE DE FRANCE, Nr. 1-2, 1974, Seiten 211-217, Paris, FR; D. CLERIN et al.: "Hétérocyclisation des alpha-acylaminoamides. III. Propriétés des amino-5 oxazoles" * Insgesamt * --- | 7,8 | |
| D,Y | EP-A-0 174 562 (HOECHST) * Insgesamt * --- | 1-8 | |
| Y | AU-A-7 997 287 (CIBA-GEIGY) * Insgesamt * & EP-A-268 554 --- | 1-8 | RECHERCHIERTE SACHGEBIETE (Int. Cl.4) |
| P,X | EP-A-0 310 555 (CIBA-GEIGY) * Insgesamt * ----- | 1-8 | |

Der vorliegende Recherchenbericht wurde für alle Patentansprüche erstellt

| Recherchenort | Abschlußdatum der Recherche | Prüfer |
|---|---|---|
| DEN HAAG | 08-08-1989 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument